# EUROPEAN PATENT SPECIFICATION

(11) **EP 2 948 413 B1**
(45) Date of publication and mention of the grant of the patent: **27.12.2017**
(21) Application number: 14701979.8
(22) Date of filing: 28.01.2014
(51) Int. Cl.: C02F 1/469, B01D 61/44, C02F 1/461, C02F 1/20

(54) **AN ELECTROLYZED WATER GENERATING METHOD AND A GENERATOR THEREFOR**
VERFAHREN ZUR ERZEUGUNG VON ELEKTROLYSIERTEM WASSER UND GENERATOR DAFÜR
PROCÉDÉ DE GÉNÉRATION D'EAU PAR ÉLECTROLYSE ET GÉNÉRATEUR ASSOCIÉ

(30) Priority: 28.01.2013 JP 2013013760
(43) Date of publication of application: 02.12.2015
(73) Proprietor: Industrie De Nora S.P.A., 20134 Milano (IT)
(72) Inventor: UNO, Masaharu, Kanagawa 252-0816 (JP); HAMAGUCHI, Katsumi, Kanagawa 252-0816 (JP)
(74) Representative: Reitstötter Kinzebach
(86) International application number: PCT/EP2014/051567
(87) International publication number: WO 2014/114806

(56) References cited:
- EP-A1- 0 826 794
- JP-A- H05 179 475
- JP-A- 2001 003 188
- JP-A- 2003 293 178
- JP-A- 2005 329 375
- KR-B1- 101 118 795
- US-A1- 2004 124 094

## Description

### TECHNICAL FIELD

The present invention relates to an electrolyzed water generating method and a generator, which generate, stably and at a high current efficiency, both acidic electrolyzed water and alkaline electrolyzed water of high quality, free from alkaline-metal chlorides with high corrosivity, such as salt.

### BACKGROUND ART

Recently the electrolyzed water generator has been high-lighted through various movements in the industries, such as: JIS Establishment for the electrolyzed water generator as household goods in 2005; articles relating to the electrolyzed water utilization in the Standards of School Lunch Hygiene Management and related manuals by the Ministry of Education, Culture, Sports, Science and Technology in 2009, and in the Instructional Materials by Japan Food Hygiene Association, associated with the Ministry of Health in 2009.

"Electrolyzed water" is a general term for the aqueous solution obtained through electrolysis treatment of tap water or thin brine at a weak DC voltage, and is roughly classified into "acidic electrolyzed water" formed at the anode and "alkaline electrolyzed water" formed at the cathode.

In general, "acidic electrolyzed water" indicates collectively acidic electrolyzed water with the pH value of 6.5 or below. It shows a strong sterilizing power widely to various pathogenic bacteria or drug-resistant bacteria thereof (such as MRSA), finding a variety of application fields including medical care, dentistry, food or agriculture. The main sterilization factor is hypochlorous acid water formed by electrolysis.

Further, "acidic electrolyzed water" is classified into strongly acidic electrolyzed water, slightly acidic electrolyzed water, and weakly acidic electrolyzed water. Electrolyzed water with hypochlorous acid as positive ingredient (concentration of available chlorine: 20-60 ppm) at pH 2.7 or below is called strongly acidic electrolyzed water (strongly acidic hypochlorous acid water). For a strongly acidic electrolyzed water generator, the application is individually authorized based on (Japanese) Pharmaceutical Affairs Law, and so far the generator is approved as a medical device (with Pharmaceutical Affairs Law revision, medical equipment production sale) for the purpose of the use mentioned below.

Strongly acidic electrolyzed water (available chlorine 40 ppm) shows antibacterial · antivirus activity (also, high norovirus inactivation effect) equal to sodium hypochlorite of a high concentration (1, 000ppm). This is because that whereas the existence rate of hypochlorous acid (HCIO), as a sterilization factor, is approximately 90% in strongly acidic electrolyzed water, sodium hypochlorite, which is alkaline, remains in less than 5% and 95% or more exist as feebly active hypochlorous acid ion (ClO⁻). However, hypochlorous acid reacts easily with organic matter and therefore, if there is much organic matter, the sterilizing power of strongly acid electrolyzed water decreases remarkably. To overcome this, a method is adopted as an effective way that an object to be sterilized is first treated in strongly alkaline electrolyzed water, where it has been revealed that a removal effect of oils, fats and protein is high, and then, treated in strongly acidic electrolyzed water. Various tests have been conducted so far about safety, from which high level is confirmed.

Slightly acidic electrolyzed water is an aqueous solution of hypochlorous acid with the pH value of 5-6.5 and available chlorine at 10-30 ppm, and it is characteristic that all the generated water is sterilization water. It shows antibacterial · antivirus activity similar to strongly acidic electrolyzed water. Safety test results are the same.

Weakly acidic electrolyzed water with a pH range filling between slightly acidic electrolyzed water and strongly acidic electrolyzed water has passed the deliberation of the Food Safety Commission. Weakly acidic electrolyzed water is recognized to have activity and safety that are equal to strongly acidic electrolyzed water or slightly acidic electrolyzed water.

On the other hand, "alkaline electrolyzed water" is composed primarily of caustic alkali generated in the cathode side simultaneously in the electrolysis. "Alkaline electrolyzed water" is roughly classified into two: strongly alkaline electrolyzed water (pH 11-11.5) and weakly alkaline electrolyzed water (pH 9-10), called alkaline ionized water, formed by electrolyzing tap water using a home electrolyzed water generator, alias alkali ion water purifier. The home electrolyzed water generator is a name of home medical equipment classified as "an appliance instrument 83 medical material generator" in the Pharmaceutical Affairs Act Enforcement Order. The effects, as mentioned below, of alkaline ionized water that received approval as a medical device have been confirmed as the results of strict comparative clinical tests. More specifically, it is effective to "chronic diarrhea, indigestion, abnormal ferment in the stomach and intestines, antacid, and hyperacidity". In addition, an improvement effect has been accepted for the constipation. It has been revised now as having "an improvement effect of the gastrointestinal symptom" with revision (2005) of the Pharmaceutical Affairs Act.

By electrolyzing aqueous solution in which electrolyte containing alkaline-metal chlorides, such as sodium chloride aqueous solution or potassium chloride aqueous solution are dissolved, in the electrolyzed water generator, acidic electrolyzed water containing hypochlorous acid water is obtained at the anode and alkaline electrolyzed water containing caustic alkali is obtained at the cathode. The electrolytic system to perform electrolysis applying sodium chloride aqueous solution and potassium chloride aqueous solution as electrolyte composes acid electrolyzed water including hypochlorous acid water having a sterilization effect for bacteria including Escherichia coli on the anode side, whereas alkaline electrolyzed water including caustic alkali having a strong detergency such as defatting and protein removal is formed on the cathode side, and widely used in the fields of food processing, agriculture, and medical · nursing-care.

For such electrolytic system to generate acidic electrolyzed water including hypochlorous acid water and alkaline electrolyzed water including caustic alkali, methods employing the two compartment cell and the three compartment cell are known.

In the present invention, hypochlorous acid water formed at the anode by electrolysis or acidic electrolyzed water including hypochlorous acid water to be formed by dissolving chlorine gas generated at the anode in the dissolution water after separation and recovery, is simply called "acidic electrolyzed water", whereas, alkaline electrolyzed water including caustic alkali is simply called "alkaline electrolyzed water".

As a method applying a two compartment cell, Patent Literature 1 describes examples. The two compartment cell has an anode and a cathode separated by a diaphragm, in which sodium chloride aqueous solution is supplied to the anode chamber and raw water such as tap water or sodium chloride aqueous solution is supplied to the cathode chamber for electrolysis operation.

It is pointed that in the acidic electrolyzed water produced in such a way, a relatively high concentration of unreacting sodium chloride remains and that such sodium chloride may precipitate after service or problems including metal corrosion of piping will occur. In such electrolyzed water generation system by the two compartments method, brine is supplied to the anode chamber to enhance electrolysis efficiency.

For this reason, the acidic electrolyzed water generated in the anode chamber, which contains not only hypochlorous acid but also sodium chloride component will cause such phenomena as chlorine gas evaporation by the equilibrium movement. Since hypochlorous acid will evaporate in a short time, it becomes difficult for the acidic electrolyzed water to secure required sterilizing power for a long time, leading to limited applications. Besides, the corrosion of the peripheral device by this sodium chloride becomes a serious obstacle to the market expansion.

Whereas, a three compartment cell, which has a configuration consisting of the anode chamber separated by an anion exchange membrane, the cathode chamber separated by a cation exchange membrane and the intermediate chamber separated by the two membranes can minimize mingling of raw material salt component into formed acidic electrolyzed water and alkaline electrolyzed water by supplying raw material brine into the intermediate chamber. Thus, the three compartment cell can solve problems so far encountered including high corrosivity and unsuitability to agricultural fields, and many companies participate in developing associated devices and many patent applications have been filed.

Representative patent literature includes Patent Literature 2 and Patent Literature 3. This method employs a three compartment cell, comprising the anode chamber, intermediate chamber and cathode chamber separated by two sheets of diaphragm having ion exchange capacity. Electrolysis is conducted in such a manner that sodium chloride aqueous solution is supplied to the intermediate chamber, and raw water free from alkaline-metal chloride is supplied to the anode chamber and the cathode chamber to compose acidic electrolyzed water at the anode and alkaline electrolyzed water at the cathode, respectively. In this method, an anion exchange membrane is applied as diaphragm to separate the anode chamber and the intermediate chamber, and a cation exchange membrane is applied as diaphragm to separate the cathode chamber and the intermediate chamber. Theoretically, only chloride ion which is necessary for acidic electrolyzed water composition migrates from the intermediate chamber to the anode chamber, and only sodium ion which is necessary for alkaline electrolyzed water composition migrates from the intermediate chamber to the cathode chamber. Therefore, it is suggested that compared with the two compartment cell, this method is advantageous in composing electrolyzed water with less residual sodium chloride, ameliorating problems of salt precipitation after use or metal corrosion by salt.

As mentioned above, the three compartment cell applies two kinds of ion exchange membrane: anion exchange membrane and cation exchange membrane to compose acidic electrolyzed water and alkaline electrolyzed water. When commercially available anion exchange membrane and cation exchange membrane are compared, it is found that the following problems occur since anion conductivity and ion selectivity of anion exchange membrane are inferior.

When, for example, electrolysis is conducted in three compartment cell in such a manner that sodium chloride aqueous solution is supplied to the intermediate chamber and raw water which does not include alkaline-metal chloride such as salt is supplied to the anode chamber and the cathode chamber, chloride ions migrate from the intermediate chamber to the anode chamber through the anion exchange membrane and at the same time, sodium ions migrate to the cathode chamber through the cation exchange membrane. At this time, the chlorine generation reaction as shown by Equation (1) progresses at the anode and formed chlorine reacts immediately with water as in Equation (2) to compose acidic electrolyzed water. However, when the supply of the chloride ions is insufficient, oxygen generation progresses competitively through electrolysis of water as shown in Equation (3). On the other hand, at the cathode, hydrogen generation progresses through electrolysis of water as in Equation (4) and sodium hydroxide water (alkaline electrolyzed water) is composed by formed hydroxyl ions and sodium ions supplied from the intermediate chamber.

2Cl⁻ → Cl₂ + 2e⁻ · · · (1)

Cl₂ + H₂O → HClO + HCI · · · (2)

2H₂O → O₂ +4H⁺ 4e⁻ · · · (3)

2H₂O + 2e⁻ → H₂ + 2OH⁻ · · · (4)

The migration speed of sodium ions penetrating a commercial cation exchange membrane is sufficiently fast, and even if the current density at the time of electrolysis is changed, for instance, from a low level of 3 A/dm² to a high level of 20 A/dm², 90% or more of applied electric current is utilized to compose alkaline electrolyzed water. However, the migration speed of chloride ions penetrating a commercial anion exchange membrane is not so high. For example, electric current utilized for composition of acidic electrolyzed water (current efficiency) is around 80 % even by the electrolysis at a low current density and decreases to around 40% at a high current density. Thus, the energy efficiency to compose hypochlorous acid water at the anode is not high, causing a problem that the higher the current density, the lower the energy efficiency.

Furthermore, for example, when electrolysis is continued by the three compartment cell while circulating and supplying sodium chloride aqueous solution to the intermediate chamber, the pH of circulating sodium chloride aqueous solution drops (acid) with time and at the same time, chlorine gas which is harmful to the human body occurs because available chlorine ingredient accumulates in sodium chloride aqueous solution, causing a safety problem of leakage outside the electrolytic system. The cause of chlorine gas generation is not clear. Hypochlorous acid and hydrochloric acid are composed by Equation (2) successively to Equation (1) and hydrogen ion is composed by the side reaction, Equation (3). The commercial anion exchange membrane applied for separation of the anode chamber and the intermediate chamber is insufficient in ion selectivity and it is expected that hypochlorous acid or hydrogen ion will move from the anode chamber to the intermediate chamber through the anion exchange membrane.

The reason why ion selectivity of a commercial anion exchange membrane assumes insufficient is the fact that even acidic electrolyzed water composed in three compartment cell gets mixed with sodium chloride of a low concentration. Anion exchange membrane does not theoretically penetrate sodium ion, which is cation, but in hypochlorous acid water prepared by using a commercial anion exchange membrane, increase of the sodium ion concentration is clearly recognized compared with raw water. On the other hand, a commercial cation exchange membrane has enough ion selectivity and, in alkaline electrolyzed water formed at the cathode, increase of the chloride ion concentration is little admitted in comparison with raw water.

In addition to the above-mentioned issues, there is another problem in commercial anion exchange membrane that since deterioration is accelerated by oxidizing agents such as hypochlorous acid, ion selectivity and anion conductivity decrease more and more when electrolysis is continued. Patent Literature 4 and Patent Literature 5 suggest as a restraint method of the anion exchange membrane deterioration that contact of oxidizers such as hypochlorous acid formed at the anode and an anion exchange membrane is physically alienated by disposing porous nonwoven fabric or a porous structure body between the anode and the anion exchange membrane. However, contact of oxidizer and the anion exchange membrane cannot be prevented completely by these methods, and cell voltage increases by inserting an insulating material between the anode and the cathode, leading to another problem of increase in electricity energy-consumption.

In this way, the utilization rate of electric energy (current efficiency) in the acidic electrolyzed water production by the electrolyzed water generator applying the conventional three compartment cell is low mainly due to applied anion exchange membrane, and a little amount of electrolyte gets mixed with produced acidic electrolyzed water inevitably. Moreover, there was a problem that the anode exchange membrane was deteriorated with a time lapse of electrolysis operation by hypochlorous acid generated at the anode. Neither a method nor a device to solve all these problems has been suggested so far.

### RELATED TECHNICAL LITERATURE

### Patent Literature

Patent Literature 1: Japanese Unexamined Patent Application Publication Hei07-214063
Patent Literature 2: Japanese Unexamined Patent Application Publication 2000-212787
Patent Literature 3: Japanese Unexamined Patent Application Publication 2009-072755
Patent Literature 4: Japanese Unexamined Patent Application Publication 2006-322053
Patent Literature 5: Japanese Unexamined Patent Application Publication 2012-110809

Methods for producing electrolyzed acidic and alkaline water are also described in documents KR 10-1118795 and US 2012/0267256.

### SUMMARY OF INVENTION

### Technical Problem

The present invention aims to provide an electrolyzed water generating method and a generator, which can overcome flaws and problems of the electrolyzed water generating method and the generator applying the conventional two compartment cell and the three compartment cell as mentioned above, can produce high-quality electrolyzed water comprising both of acidic electrolyzed water and alkaline electrolyzed water, free from highly corrosive alkaline-metal chloride like salt at a high current efficiency, can control the pH of acidic electrolyzed water, and can operate stably for a long time with high durability.

### Solution to Problem

The above mentioned problem is solved with a method according to the claims 1-5 and a generator according to the claims 6-9.

### Advantageous Effects of Invention

According to the electrolyzed water generating method and the generator which the present invention suggests, electrolysis is performed with the raw water free from alkaline-metal chloride being supplied to the cathode chamber of the two compartment cell divided by the cation exchange membrane. Then, alkaline electrolyzed water almost free from alkaline-metal chloride can be produced on the cathode side, at a high current efficiency. Whereas, on the anode side, anodic electrolyte comprising aqueous solution with dissolved alkaline-metal chloride is circulated from the storage tank of anodic electrolyte which retains anodic electrolyte, producing chlorine containing gas of high concentration at a high current efficiency. The chlorine containing gas of high concentration is collected at the gas-liquid separator, separated from anodic electrolyte comprising an aqueous solution with dissolved alkaline-metal chloride and is come in contact with dissolution fluid which does not dissolve alkaline-metal chloride, to be dissolved at the dissolver of chlorine gas. In such a way, acidic electrolyzed water practically free from alkaline-metal chloride can be produced efficiently. In addition, the present invention can improve durability because the two compartment cell comprising the anode, cathode and cation exchange membrane only with a high durability is used, without using an anion exchange membrane with many problems including durability.

Moreover, the present invention can produce any arbitrarily desired strength of strongly acidic electrolyzed water, weakly acidic electrolyzed water, or slightly acidic electrolyzed water through regulating the pH value of acidic electrolyzed water free from alkaline-metal chloride by adding electrolytically produced alkaline electrolyzed water under control of the flow rate when the gas separated and collected from the anodic electrolyte is come in contact with the dissolution fluid to be dissolved.

Furthermore, when chlorine comes to be released from the anodic electrolyte retained in the storage tank of anodic electrolyte and gradually pervades in the storage tank of anodic electrolyte if electrolysis is continued, the present invention can prevent chlorine from leaking and promote effective utilization of chlorine gas by letting chlorine containing gas in the storage tank of anodic electrolyte join chlorine containing gas which has evolved at the anode and sending it to the dissolver of chlorine gas.

Furthermore, in the electrolytic cell by the present invention, the applied cathode is porous body and is disposed in close contact with the cation exchange membrane. The anode chamber is configured to have a higher back pressure than the cathode chamber so that the cation exchange membrane is pushed to the porous cathode, which can maintain a low cell voltage and remarkably reduce power consumption by synergy effect with the enhanced current efficiency, in comparison with the conventional three compartment cell.

Furthermore, according to the present invention, chlorine generation by Equation (1) is promoted effectively by electrolysis applying anodic electrolyte with alkaline-metal chloride dissolved at 10 wt% or more. Generated chlorine first reacts with anodic electrolyte, as shown in Equation (2) and accumulated as hypochlorous acid and hydrochloric acid. When chlorine dissolved in anodic electrolyte reaches saturation, chlorine comes to occur as gas.

Furthermore, according to the present invention, chlorine can be prevented from being released outside the present generator by controlling the contact and dissolution time of chlorine containing gas and the dissolution fluid to 0.05 seconds or more per 1 ml of the gas.

Furthermore, according to the present invention, portion of raw water in the inlet tube of raw water to supply raw water free from alkaline-metal chloride to the cathode chamber can be branched and linked to the inlet tube of dissolution fluid to supply dissolution fluid to the dissolver of chlorine gas. Thus, the branched raw water can be used as dissolution fluid, leading to effective utilization of the facilities. However, since the purpose of raw water is different from dissolution fluid, as to be mentioned, individual aqueous solution may be better to be used in some cases.

### BRIEF DESCRIPTION OF DRAWINGS

Fig.1 A flow diagram showing an example of electrolyzed water generator by the present invention;
Fig.2 A flow diagram showing an example of conventional electrolyzed water generator

### DESCRIPTION OF EMBODIMENT

The following describes the embodiment of the present invention in reference to the figures.

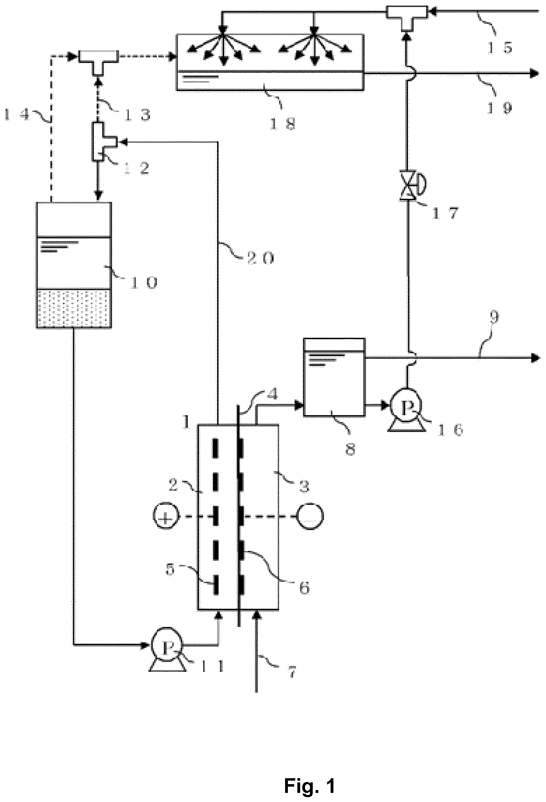

Fig.1 shows an example of the electrolyzed water generator by the present invention, comprising the two compartment cell 1, the anode chamber 2, the cathode chamber 3, the cation exchange membrane 4, the anode 5, the cathode 6, the Inlet tube of raw water 7, the storage tank of alkaline electrolyzed water 8, the outlet tube of alkaline electrolyzed water 9, the storage tank of anodic electrolyte 10, the circulator 11, the gas-liquid separator 12, the anodic gas tube 13, the chlorine gas tube 14, the inlet tube of dissolution fluid 15, the alkaline electrolyzed water pump 16, the flow control valve 17, the dissolver of chlorine gas 18, the outlet tube of acidic electrolyzed water 19, and the outlet tube of anodic electrolyte 20.

In the present invention, the anodic electrolyte comprising aqueous solution in which alkaline-metal chloride like salt is dissolved is supplied to the anode chamber 2 of the two compartment cell 1 separated into two compartments: the anode chamber 2 accommodating the anode 5 and the cathode chamber 3 accommodating the cathode 6 by the cation exchange membrane 4 from the storage tank of anodic electrolyte 10 which store the anodic electrolyte using the circulator 11, and electrolysis is performed while raw water free from alkaline-metal chloride like salt is being supplied from the inlet tube of raw water 7 to the cathode chamber 3. By electrolysis operation, alkaline electrolyzed water free from alkaline-metal chloride is produced at the cathode chamber 3. Produced alkaline electrolyzed water is discharged from the outlet tube of alkaline electrolyzed water 9 through the storage tank of alkaline electrolyzed water 8.

Chlorine containing gas is generated in the anode chamber 2, and separated from anodic electrolyte at the gas-liquid separator 12 and thus collected gas is sent to the dissolver of chlorine gas 18 through the anodic gas tube 13. Dissolution fluid free from alkaline-metal chloride like salt is supplied to the dissolver of chlorine gas 18 from the inlet tube of dissolution fluid 15 and acidic electrolyzed water free from alkaline-metal chloride like salt is generated in the dissolver of chlorine gas 18. Generated acidic electrolyzed water is discharged through the outlet tube of acidic electrolyzed water 19.

On the other hand, the anodic electrolyte separated by the gas-liquid separator 12 is circulated to the storage tank of anodic electrolyte 10.

The alkaline-metal chloride contained in the anodic electrolyte in the storage tank of anodic electrolyte 10 is partially decomposed to evolve chlorine gas. In order to eliminate the negative effect of gas leakage occurring from the chlorine gas and to utilize it effectively, the chlorine gas evolved in the storage tank of anodic electrolyte 10 is sent to the dissolver of chlorine gas 18 via the chlorine gas tube 14 to be used to produce acidic electrolyzed water.
Portion of raw water can be used as the dissolution fluid to be supplied to the dissolver of chlorine gas 18 via a tube (not illustrated) branched from the Inlet tube of raw water 7.
Alkaline electrolyzed water electrolytically produced in the cathode chamber 3 can be added to the dissolution fluid in the dissolver of chlorine gas 18 under the control of flow rate by the flow control valve 17 through the storage tank of alkaline electrolyzed water 8 by the alkaline electrolyzed water pump16. In such a way, the pH value of acidic electrolyzed water produced in the dissolver of chlorine gas 18 can be controlled to a desired value.
For alkaline-metal chloride to be used for anodic electrolyte, LiCl, NaCl, and KCI are exemplified, among which NaCl and KCI can be preferably applied. As raw water, there is available soft water prepared by removing Ca ion and Mg ion contained in well water and city water, ion exchanged water prepared by further removing other cation and anion, and pure water prepared by removing even organic component.
When electrolysis is conducted while anodic electrolyte containing one or more kinds of these alkaline-metal chlorides is being supplied to the anode chamber 2, the chlorine generation reaction shown in Equation (1) and oxygen generation reaction in Equation (3) progress competitively at the anode 5. The present invention lets chlorine generation in Equation (1) progress effectively by controlling the concentration of alkaline-metal chloride in the aqueous solution supplied to the anode chamber 2 to 10 wt% or more. Evolved chlorine first reacts with anodic electrolyte as shown in Equation (2) and accumulated as hypochlorous acid and hydrochloric acid and when the dissolved amount of chlorine in the anodic electrolyte reaches saturation, chlorine evolves as gas. In order to generate chlorine gas effectively, it is effective either to let anodic electrolyte circulate until the dissolved amount of chlorine reaches saturation or to lower the saturation concentration of chlorine dissolution by lowering the pH value with HCl added to the anodic electrolyte.

When electrolysis is conducted while the above-exemplified raw water is being supplied to the cathode chamber 3, hydroxyl ion occurs from electrolytic reaction of water shown in Equation (4) at the cathode 6 and alkaline electrolyzed water in which cation from the anode chamber 2 penetrated through the cation exchange membrane 4 is the counter ion is produced and discharged from the outlet tube of alkaline electrolyzed water 9 through the storage tank of alkaline electrolyzed water 8. The pH of the alkaline electrolyzed water is 8 or more, though it varies with the flow rate of raw water or the current density at the time of electrolysis. When hardness ingredients such as Ca ion and Mg ion are contained in raw water, scaling develops on the surface of the cathode 6, inside of the cathode chamber 3 and internal of the outlet tube of alkaline electrolyzed water 9. Such problems are easy to occur, in a long time of electrolysis, that the cathode reaction is inhibited or that the flow volume of alkaline electrolyzed water decreases. To suppress such a malfunction, it is particularly preferable to use soft water, ion exchanged water or pure water for the raw water to be supplied to the cathode chamber 3.

Since raw water having a low conductivity is supplied to the cathode chamber 3, the cell voltage at the time of electrolysis becomes remarkably high if a cavity exists between the cation exchange membrane 4 and the cathode 6, leading to a problem of increased power consumption. Then, to cope with this, the cathode 6 is made with porous materials such as mesh, punched plate, and foamed body and is disposed to be closely attached to the cation exchange membrane 4, and the back pressure of the anode chamber 2 is made larger than that of the cathode chamber 3, so that the electrolytic voltage at the time of electrolysis is kept low by the configuration that the cation exchange membrane 4 is pushed to the porous cathode 6. For the anode chamber 2, increase of cell voltage at the time of electrolysis is small even if a cavity exists between the cation exchange membrane 4 and the anode 5, since the anodic electrolyte of the high conductivity is supplied. To keep the back pressure of the anode chamber 2 higher than that of the cathode chamber 3, the height of the gas-liquid separator 12 located above the anode chamber 2 is kept greater than the height of the storage tank of alkaline electrolyzed water 8 and the outlet tube of alkaline electrolyzed water 9 located downstream of the cathode chamber 3.
Chlorine containing gas evolved at the anode 5 is supplied to the gas-liquid separator 12 together with anodic electrolyte and collected gas only moves to the anodic gas tube 13 and anodic electrolyte is returned to the storage tank of anodic electrolyte 10. As mentioned above, chlorine dissolves in the anodic electrolyte up to nearly the saturation concentration. When electrolysis is continued, chlorine comes to be released from the anodic electrolyte accumulated in the storage tank of anodic electrolyte 10, and gradually the tank becomes full of chlorine, and eventually a problem occurs in the security that chlorine leaks outside the tank. Such chlorine leakage problem can be prevented in such a way that chlorine containing gas in the storage tank of anodic electrolyte 10 is induced to the chlorine gas tube 14 and let it join the gas containing chlorine evolved at the anode 5 and transferred to the anodic gas tube 13.
Chlorine in the gas comes in contact with the dissolution fluid in the dissolver of chlorine gas 18 and dissolves to become acidic electrolyzed water by the reaction shown in Equation (2). Whereas, when the full amount of chlorine supplied to the dissolver of chlorine gas 18 does not contact the dissolution fluid and is not dissolved, undissolved chlorine is released outside the system, causing a problem in the security. To prevent this problem from occurring, it is necessary to supply the dissolution fluid of the quantity enough to dissolve the chlorine supplied to the dissolver of chlorine gas 18. In addition, it is preferable for the dissolver of chlorine gas 18 to have means to promote contact and dissolution of chlorine such as a sprinkler, a gas diffuser, an external stirrer, a static stirrer, and a scrubber. Moreover, release of chlorine outside the system can be prevented by controlling the contact time for dissolution of the chlorine containing gas evolved from the electrolysis with the dissolution fluid to 0.05 seconds or more per 1 ml of the gas.

In the present generator, the dissolution fluid to be used for manufacturing acidic electrolyzed water may or may not be the same as the raw water to be used for manufacturing alkaline electrolyzed water. In the reaction of water and chlorine shown in Equation (2), hydrochloric acid is by-produced besides hypochlorous acid, and then, the acidic electrolyzed water by the present generator tends to become acid. As mentioned above, as the raw water supplied to the cathode chamber 23, use of soft water, ion exchanged water or the pure water is preferable to control scaling of Ca ion and Mg ion. Whereas, for the dissolution fluid to be used for manufacturing hypochlorous acid water, waters containing Ca ion and Mg ion, such as well water or city water can be used without problem.

In the present generator, the concentration of hypochlorous acid in the acidic electrolyzed water can be regulated by the supply volume of dissolution fluid and that of chorine containing gas evolved from the anode 5. However, the pH value lowers together with the concentration of hypochlorous acid, because hydrochloric acid is by-produced as shown in Equation (2). As mentioned above, hypochlorous acid water in acidic electrolyzed water has a strong oxidation power, and is utilized for the sterilization use of Escherichia coli and bacteria. The sterilizing power, however, varies with the pH value and around pH 6 is known to be strong. In the domain of low pH, hypochlorous acid becomes equilibrium with chlorine, generating a risk that chlorine gas is released from hypochlorous acid water in acidic electrolyzed water. Therefore, in the present generator, produced alkaline electrolyzed water is retained once in the storage tank of alkaline electrolyzed water 8, as shown in Fig.1, and the pH value of generated acidic electrolyzed water can be regulated by mixing a suitable amount of alkaline electrolyzed water with the dissolution fluid by using the alkaline electrolyzed water pump16 and the flow control valve 17.

### EXAMPLE

The following explains examples of the production of hypochlorous acid water and alkaline electrolyzed water using the electrolyzed water generator by the present invention, but the present invention is not limited to these embodiments.

### Example 1 (not according to the invention)

In the electrolytic system as shown in Fig.1, the two compartment cell 1 comprised the electrodes (JL-510 manufactured by Permelec Electrode Ltd.) of the anode 5 and the cathode 6 prepared in such a manner that platinum catalyst was coated on the mesh-shape titanium substrate by the thermal decomposition method with 60 cm² of projected area and the cation exchange membrane 4 (Nafion (registered trade mark) N-115 manufactured by Du Pont) which separated the anode chamber 2 and the cathode chamber 3. The cation exchange membrane 4 was disposed so that the respective electrode came in contact with the membrane on each side. The gas-liquid separator 12 was placed 5 cm above the anode chamber 2 and was regulated so that the back pressure of 50 mmH₂O was applied to the anode chamber 2, and the outlet tube of alkaline electrolyzed water 9 also was placed 5 cm above the cathode chamber 3 and was regulated so that the back pressure of 50 mmH₂O was applied to the cathode chamber 3.

In Example 1, the chlorine gas tube 14 above the storage tank of anodic electrolyte 10 in Fig.1 was not installed and the anodic gas tube 13 was directly connected to the dissolver of chlorine gas 18. To the dissolver of chlorine gas 18, the dissolution fluid from the inlet tube of dissolution fluid 15 and the alkaline electrolyzed water from the storage tank of alkaline electrolyzed water 8 were mixed and sprayed from the upper part of the dissolver of chlorine gas 18.

The anodic electrolyte which was sodium chloride aqueous solution of about 8 wt% was circulated between the storage tank of anodic electrolyte 10 and the anode chamber 2 by the circulator 11. While soft water was being supplied as raw water to the cathode chamber 3 at the flow rate of 1 L/min.., electrolysis was carried out using electric current applied at 6A to the anode 5 and the cathode 6. To the dissolver of chlorine gas 18, tap water was supplied at the flow rate of 1 L/min. as the dissolution fluid, which was made contact with chlorine containing gas supplied through the anodic gas tube 13 for dissolution to compose acidic electrolyzed water. The capacity of the dissolver of chlorine gas 18 was designed so that the contact and dissolution time of the dissolution fluid and chlorine containing gas was controlled to one second.

In one hour from the start of electrolysis, measurement of cell voltage was 28 V, the concentration of available chlorine in hypochlorous acid water sampled from the outlet tube of acidic electrolyzed water 19 was 108 mg/L as chlorine, and the pH was 2.7 and increment of the sodium chloride density was 4 mg/L. The pH value of alkaline electrolyzed water sampled from the outlet tube of alkaline electrolyzed water 9 was 11.6, and the increment of sodium chloride density was 1 mg/L. Around the exit of the outlet tube of acidic electrolyzed water 19 and the storage tank of anodic electrolyte 10, chlorine smell was felt just a little, but not to a problematic level. The quantity of chlorine generation that was calculated from the quantity of electricity applied was about 40 NmL/min. and the contact and dissolution time of the dissolution fluid with chlorine containing gas at the dissolver of chlorine gas 18 was estimated to be 0.025 seconds per 1 mL of the gas.

### Example 2 (not according to the invention)

Under the same conditions as with Example 1, electrolysis was performed using the electrolytic system mentioned in Example 1, adding arbitrary quantity of alkaline electrolyzed water formed by electrolysis to the dissolution fluid supplied to the dissolver of chlorine gas 18. In one hour from the start of electrolysis, measurement of cell voltage was 28 V, and when the added quantity of alkaline electrolyzed water to the dissolution fluid was changed, the quantity of generated acidic electrolyzed water, the concentration of available chlorine and pH value, the quantity of generated alkaline electrolyzed water and pH value were as per Table 1. Adjustment of the pH value was possible by controlling the addition of the alkaline electrolyzed water to the dissolution fluid.

| quantity of dissolution fluid (L/min) | added quantity of alkaline electrolyzed water (L/min) | acidic electrolyzed water | | | alkaline electrolyzed water | |
|---|---|---|---|---|---|---|
| | | generated quantity (L/min) | concentration of available chlorine (mg/L) | pH | generated quantity (L/min) | pH |
| 1 | 0 | 1 | 108 | 2.7 | 1 | 11.6 |
| 0.8 | 0.2 | 1 | 108 | 3.0 | 0.8 | 11.6 |
| 0.7 | 0.3 | 1 | 108 | 3.2 | 0.7 | 11.6 |
| 0.6 | 0.4 | 1 | 108 | 3.7 | 0.6 | 11.6 |
| 0.5 | 0.5 | 1 | 108 | 6.2 | 0.5 | 11.6 |
| 0.4 | 0.6 | 1 | 108 | 7.0 | 0.4 | 11.6 |
| 0.3 | 0.7 | 1 | 108 | 7.6 | 0.3 | 11.6 |
| 0.2 | 0.8 | 1 | 108 | 8.0 | 0.2 | 11.6 |
| 0 | 1.0 | 1 | 108 | 8.7 | - | - |

### Example 3 (not according to the invention)

With the same electrolytic system described in Example 1, electrolysis operation was performed by the same method as Example 1, using the same electrolyzed water generator as Example 1, except that the chlorine gas tube 14 was connected to the upper part of the storage tank of anodic electrolyte 10 and connected to the dissolver of chlorine gas 18 after the chlorine gas tube 14 and the anodic gas tube 13 were joined together in Example 3.

In one hour from the start of electrolysis, measurement of cell voltage was 28 V, the concentration of available chlorine in acidic electrolyzed water sampled from the outlet tube of acidic electrolyzed water 19 was 108 mg/L as chlorine, and the pH was 2.7 and increment of the sodium chloride density was 4 mg/L. The pH value of alkaline electrolyzed water sampled from the outlet tube of alkaline electrolyzed water 9 was 11.6, and the increment of sodium chloride density was 1 mg/L. Chlorine smell was felt just a little around the exit of the outlet tube of acidic electrolyzed water 19, but no chlorine smell was felt around the storage tank of anodic electrolyte 10.

### Example 4

Electrolysis was started using the same electrolytic system and method as Example 3, except that the outlet tube of alkaline electrolyzed water 9 is disposed 5 cm above the cathode chamber 3 as with Example 1, and the back pressure to the cathode chamber 3 was being regulated to 50 mmH₂O, the gas-liquid separator 12 was disposed 30 cm above the anode chamber 2 and the back pressure to the anode chamber 2 was being regulated to 300 mmH₂O in Example 4. Thus, in Example 4, the cation exchange membrane was pushed to the porous cathode by increasing the back pressure of the anode chamber larger than that of the cathode chamber.

In one hour from the start of electrolysis, measurement of cell voltage was 2.8 V, the concentration of available chlorine in acidic electrolyzed water sampled from the outlet tube of acidic electrolyzed water 19 was 108 mg/L as chlorine, and the pH was 2.7 and increment of the sodium chloride density was 4 mg/L. The pH value of alkaline electrolyzed water sampled from the outlet tube of alkaline electrolyzed water 9 was 11.6, and the increment of sodium chloride density was 1 mg/L.

### Example 5

Electrolysis operation was performed by the same electrolytic system and by the same method as those described in Example 4, except that the anodic electrolyte was sodium chloride aqueous solution of 30 wt% in Example 5.

In one hour from the start of electrolysis, measurement of cell voltage was 2.6 V, the concentration of available chlorine in acidic electrolyzed water sampled from the outlet tube of acidic electrolyzed water 19 was 113 mg/L as chlorine, the pH was 2.8 and the increment of the sodium chloride density was 4 mg/L. The pH value of alkaline electrolyzed water sampled from the outlet tube of alkaline electrolyzed water 9 was 11.7, and the increment of sodium chloride density was 1 mg/L.

### Example 6

Electrolysis operation was performed by the same electrolytic system and by the same method as those described in Example 5, except that the contact and dissolution time of the dissolution fluid with the chlorine containing gas in the dissolver of chlorine gas 18 was two seconds in Example 6.

In one hour from the start of electrolysis, measurement of cell voltage was 2.6 V, the concentration of available chlorine in acidic electrolyzed water sampled from the outlet tube of acidic electrolyzed water 19 was 120 mg/L as chlorine, the pH was 2.7 and the increment of the sodium chloride density was 4 mg/L. The pH value of alkaline electrolyzed water sampled from the outlet tube of alkaline electrolyzed water 9 was 11.7, and the increment of sodium chloride density was 1 mg/L. No chlorine smell was felt around the storage tank of anodic electrolyte 10 and the exit of the outlet tube of acidic electrolyzed water 19.

The contact and dissolution time of the dissolution fluid with chlorine containing gas was estimated to be 0.05 seconds per 1 mL of the gas.

### Comparative Example 1

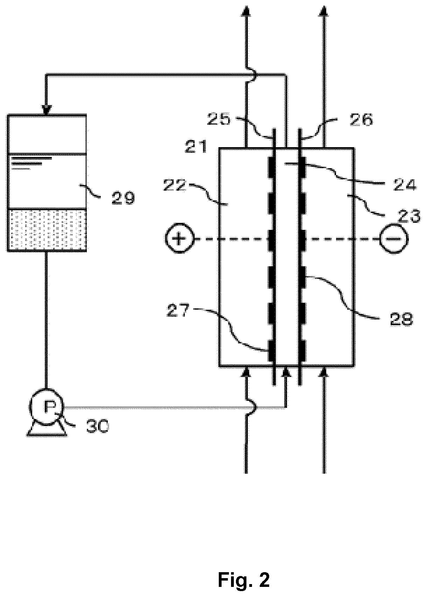

Fig.2 is a conventional type of electrolyzed water generator using a three compartment cell, comprising the three compartment cell 21, the anode chamber 22, the cathode chamber 23, the intermediate chamber 24, the anion exchange membrane 25, the cation exchange membrane 26, the anode 27, the cathode 28, the storage tank of intermediate chamber electrolyte 29, and the circulator 30. In the electrolyzed water generator shown in Fig.2, the three compartment cell 21 is separated by the anion exchange membrane 25 (Neosepta (registered trade mark) AHA manufactured by Tokuyama Corporation) into the anode chamber 22 and the intermediate chamber 24 and further separated by the cation exchange membrane 26 (Nafion (registered trade mark) N-115 manufactured by Du Pont) into the cathode chamber 23 and the intermediate chamber 24. The electrodes (JL-510 manufactured by Permelec Electrode) of the anode 27 and the cathode 28, each made of mesh-shape titanium substrate with the projected area of 60 cm2 coated with platinum catalyst by thermal decomposition method were disposed in the anode chamber 22 and the cathode chamber 23, respectively.

The intermediate chamber solution which was sodium chloride aqueous solution of about 30 wt% was circulated by the circulator 30 between the storage tank of intermediate chamber electrolyte 29 and the intermediate chamber 24. Soft water, as raw water, was supplied to the cathode chamber 23 at a flow rate of I L/min. and tap water, as raw water, was supplied to the anode chamber 22, and electrolysis was carried out by electric current applied at 6A to the anode 27 and the cathode 28.

In one hour from the start of electrolysis, measurement of cell voltage was 6.2 V, the concentration of available chlorine in acidic electrolyzed water produced at the anode 27 was 71 mg/L as chlorine, and the pH was 2.6 and the increment of the sodium chloride density was 47 mg/L. The pH value of alkaline electrolyzed water produced at the cathode 28 was 11.7, and the increment of sodium chloride density was 1 mg/L.

### INDUSTRIAL APPLICABILITY

The electrolyzed water generating method and the generator by the present invention can minimize the mingling of raw materials salt ingredient into generated acidic electrolyzed water and alkaline electrolyzed water and therefore, can widely apply in the industry associated with high corrosivity and in agricultural fields.

### REFERENCE SIGNS LIST

- 1: Two compartment cell
- 2: Anode chamber
- 3: Cathode chamber
- 4: Cation exchange membrane
- 5: Anode
- 6: Cathode
- 7: Inlet tube of raw water
- 8: Storage tank of alkaline electrolyzed water
- 9: Outlet tube of alkaline electrolyzed water
- 10: Storage tank of anodic electrolyte
- 11: Circulator
- 12: Gas-liquid separator
- 13: Anodic gas tube
- 14: Chlorine gas tube
- 15: Inlet tube of dissolution fluid
- 16: Alkaline electrolyzed water pump
- 17: Flow control valve
- 18: Dissolver of chlorine gas
- 19: Outlet tube of acidic electrolyzed water
- 20: Outlet tube of anodic electrolyte
- 21: Three compartment cell
- 22: Anode chamber
- 23: Cathode chamber
- 24: Intermediate chamber
- 25: Anion exchange membrane
- 26: Cation exchange membrane
- 27: Anode
- 28: Cathode
- 29: Storage tank of intermediate chamber electrolyte
- 30: Circulator

## Claims

1. An electrolyzed water generating method, comprising the steps of:
anodic electrolyte comprising aqueous solution with dissolved alkaline-metal chloride is supplied and circulated from a storage tank (10) of anodic electrolyte which retains anodic electrolyte to an anode chamber (2) of a two compartment cell (1) separated by a cation exchange membrane (4) into two chambers of an anode chamber (2) accommodating an anode (5) and a cathode chamber (3) accommodating a cathode (6), wherein said cathode (6) is made with porous materials and is disposed to be closely attached to said cation exchange membrane (4), and wherein the back pressure of said anode chamber (2) is made larger than that of said cathode chamber (3), raw water free from alkaline-metal chloride is supplied to the cathode chamber (3), wherein said raw water free from alkaline-metal chloride consists of soft water prepared by removing Ca ion and Mg ion contained in well water and city water or ion exchanged water prepared by further removing other cation and anion or pure water prepared by further removing organic components,
and electrolysis is carried out, whereby
alkaline electrolyzed water free from alkaline-metal chloride at the cathode chamber (3) is produced and simultaneously,
chlorine containing gas is produced at the anode chamber (2), after the gas is separated and collected from the anodic electrolyte, said gas is allowed to come in contact with dissolution fluid free from alkaline-metal chloride to be dissolved, and
acidic electrolyzed water free from alkaline-metal chloride is produced.

2. The electrolyzed water generating method as in claim 1, wherein when the gas separated and collected from anodic electrolyte is come in contact with dissolution fluid to be dissolved, electrolytically produced alkaline electrolyzed water is added to the dissolution fluid at a regulated flow rate for controlling the pH of the acidic electrolyzed water free from alkaline-metal chloride.

3. The electrolyzed water generating method as in claim 1 or claim 2, wherein after chlorine containing gas evolved from the storage tank of anodic electrolyte is collected and mixed with chlorine containing gas evolved in the anode chamber, the mixed chlorine containing gas is come in contact with the dissolution fluid to be dissolved to produce acidic electrolyzed water free from alkaline-metal chloride.

4. The electrolyzed water generating method as in any one of claims 1-3, wherein electrolysis is carried out by applying anodic electrolyte in which alkaline-metal chloride is dissolved at 10 wt% or more.

5. The electrolyzed water generating method as in any one of claims 1-4, wherein the contact and dissolution time of the electrolytically generated chlorine containing gas with the dissolution fluid is 0.05 seconds or more per 1 ml of the gas.

6. An electrolyzed water generator to produce simultaneously acidic electrolyzed water free from alkaline-metal chloride and alkaline electrolyzed water free from alkaline-metal chloride by a two compartment cell (1), comprising:
a two compartment cell (1) divided into two compartments of an anode chamber (2) accommodating an anode (5) and a cathode chamber (3) accommodating a cathode (6) by an cation exchange membrane (4), wherein said cathode (6) is made with porous materials and is disposed to be closely attached to said cation exchange membrane (4), and
wherein, in operation, the back pressure of said anode chamber (2) is larger than that of said cathode chamber (3), a storage tank (10) of anodic electrolyte to retain anodic electrolyte comprising an aqueous solution in which alkaline metal chloride is dissolved,
a circulator (11) circulating anodic electrolyte in the storage tank (10) of anodic electrolyte to the anode chamber (2), an outlet tube (20) of anodic electrolyte for discharging chlorine containing gas evolved in the anode chamber (2) and anodic electrolyte with dissolved gas thereof from the anode chamber (2), a gas-liquid separator (12) which separates chlorine containing gas from the outlet tube (20) of anodic electrolyte and anodic electrolyte with the dissolved gas,
a dissolver (18) of chlorine gas to produce acidic electrolyzed water free from alkaline-metal chloride by making the gas, after separated and collected from anodic electrolyte at the gas-liquid separator (12), contact with dissolution fluid free from alkaline-metal chloride,
an inlet tube (15) of dissolution fluid free from alkaline-metal chloride to supply dissolution fluid to the dissolver of chlorine gas,
an inlet tube (7) of raw water to supply raw water free from alkaline-metal chloride to the cathode chamber (3), and
an outlet tube (9) of alkaline electrolyzed water to discharge alkaline electrolyzed water generated in the cathode chamber from the cathode chamber (3),
whereby the height of the gas-liquid separator (12) located above the anode chamber (2) is kept greater than the height of the storage tank of alkaline electrolyzed water (8) and the outlet tube of electrolyzed water (9) located downstream of the cathode chamber (3).

7. The electrolyzed water generator as in claims 6, wherein the pH of the acidic electrolyzed water free from alkaline-metal chloride is regulated by adding electrolytically produced alkaline electrolyzed water at a controlled flow rate to the dissolver of chlorine gas, where the acidic electrolyzed water free from alkaline-metal chloride is produced with the gas separated and collected from the anodic electrolyte at the gas-liquid separator, said gas is allowed to come in contact with dissolution fluid to be dissolved.

8. The electrolyzed water generator as in claim 6 or claim 7, wherein acidic electrolyzed water free from alkaline-metal chloride is produced by collecting chlorine containing gas evolved in the storage tank of anodic electrolyte, mixing it with chlorine containing gas evolved in the anode chamber, and bringing the mixed gases into contact with dissolution fluid to be dissolved.

9. The electrolyzed water generator as in any one of claims 6-8, wherein portion of raw water in the inlet tube to supply raw water free from alkaline-metal chloride to the cathode chamber is branched and linked to the inlet tube to supply dissolution fluid to the dissolver of chlorine gas, and branched raw water is utilized as dissolution fluid.

## Patentansprüche

1. Verfahren zum Erzeugen von elektrolysiertem Wasser, umfassend die folgenden Schritte:
Zuführen und Zirkulieren von anodischem Elektrolyt, umfassend eine wässrige Lösung mit einem gelösten Alkalimetall-Chlorid, aus einem Vorratsbehälter (10) für anodischen Elektrolyt, der anodischen Elektrolyt enthält, in eine Anodenkammer (2) einer Zweikammerzelle (1), welche durch eine Kationenaustauschmembran (4) in zwei Kammern unterteilt wird, nämlich eine Anodenkammer (2), in der eine Anode (5) angeordnet ist, und eine Kathodenkammer (3), in der eine Kathode (6) angeordnet ist, wobei die Kathode (6) aus porösen Materialien gebildet und so angeordnet ist, dass sie eng an der Kationenaustauschmembran (4) angebracht ist, und wobei der Gegendruck der Anodenkammer (2) größer als derjenige der Kathodenkammer (3) eingestellt ist,
Zuführen von Rohwasser, das frei von Alkalimetall-Chlorid ist, in die Kathodenkammer (3), wobei das Alkalimetall-Chlorid-freie Rohwasser aus weichem Wasser besteht, erhalten durch Entfernen von Ca- und Mg-Ionen, die in Quellwasser und Frischwasser enthalten sind, oder lonenaustausch-Wasser, erhalten durch weiteres Entfernen anderer Kationen und Anionen, oder reines Wasser, erhalten durch weiteres Entfernen organischer Komponenten, und
Durchführen einer Elektrolyse, wobei
in der Kathodenkammer (3) von Alkalimetall-Chlorid freies alkalisches elektrolysiertes Wasser hergestellt wird und gleichzeitig
in der Anodenkammer (2) chlorhaltiges Gas hergestellt wird,
Herstellen von saurem elektrolysiertem Wasser frei von Alkalimetall-Chlorid, indem das Gas, nachdem es von dem anodischen Elektrolyt getrennt und gesammelt wurde, mit einem Lösungsfluid, das frei von Alkalimetall-Chlorid ist, in Kontakt gebracht wird, um es darin zu lösen.

2. Verfahren zum Erzeugen von elektrolysiertem Wasser gemäß Anspruch 1, wobei, wenn das von dem anodischen Elektrolyt getrennte und entnommene Gas mit dem Lösungsfluid in Kontakt gebracht wurde, um es darin zu lösen, elektrolytisch hergestelltes alkalisches elektrolysiertes Wasser mit einer geregelten Flussrate zu dem Lösungsfluid hinzugefügt wird, um den pH-Wert des Alkalimetall-Chlorid-freien, sauren elektrolysierten Wassers einzustellen.

3. Verfahren zum Erzeugen von elektrolysiertem Wasser gemäß Anspruch 1 oder Anspruch 2, wobei, nachdem in dem Vorratsbehälter für anodischen Elektrolyt entwickeltes chlorhaltiges Gas entnommen und mit in der Anodenkammer entwickeltem chlorhaltigem Gas gemischt wurde, das gemischte chlorhaltige Gas mit dem Lösungsfluid in Kontakt gebracht wird, um es darin zu lösen, um saures elektrolysiertes Wasser frei von Alkalimetall-Chlorid herzustellen.

4. Verfahren zum Erzeugen von elektrolysiertem Wasser gemäß einem der Ansprüche 1 bis 3, wobei die Elektrolyse durch Verwenden eines anodischen Elektrolyten ausgeführt wird, in dem 10 Gew.-% oder mehr Alkalimetall-Chlorid gelöst ist.

5. Verfahren zum Erzeugen von elektrolysiertem Wasser gemäß einem der Ansprüche 1 bis 4, wobei die Kontakt- und Lösungsdauer des elektrolytisch erzeugten chlorhaltigen Gases mit dem Lösungsfluid 0,05 Sekunden oder mehr pro 1 ml Gas beträgt.

6. Vorrichtung zum Erzeugen von elektrolysiertem Wasser, zum gleichzeitigen Herstellen von saurem elektrolysiertem Wasser frei von Alkalimetall-Chlorid und alkalischem elektrolysiertem Wasser frei von Alkalimetall-Chlorid durch eine Zweikammerzelle (1), umfassend:
eine Zweikammerzelle (1), die durch eine Kationenaustauschmembran (4) in zwei Kammern unterteilt wird, nämlich eine Anodenkammer (2), in der eine Anode (5) angeordnet ist, und eine Kathodenkammer (3), in der eine Kathode (6) angeordnet ist, wobei die Kathode (6) aus porösen Materialien gebildet und so angeordnet ist, dass sie eng an der Kationenaustauschmembran (4) angebracht ist, und wobei beim Betrieb der Gegendruck der Anodenkammer (2) größer ist als derjenige der Kathodenkammer (3),
einen Vorratsbehälter (10) für anodischen Elektrolyt zur Aufnahme eines anodischen Elektrolyten, der eine wässrige Lösung umfasst, in der Alkalimetall-Chlorid gelöst ist,
einen Zirkulator (11), der anodischen Elektrolyt in dem Vorratsbehälter (10) für anodischen Elektrolyt zu der Anodenkammer (2) zirkulieren lässt,
eine Entnahmeleitung (20) für anodischen Elektrolyt zum Entnehmen von in der Anodenkammer (2) entwickeltem chlorhaltigem Gas und anodischem Elektrolyt mit gelöstem Gas davon aus der Anodenkammer (2),
einen Gas-Flüssigkeitsabscheider (12), der chlorhaltiges Gas aus der Entnahmeleitung (20) für anodischen Elektrolyt von anodischem Elektrolyt mit dem gelösten Gas abscheidet,
einen Auflöser (18) für Chlorgas zum Herstellen von saurem elektrolysiertem Wasser frei von Alkalimetall-Chlorid durch Inkontaktbringen des Gases mit Lösungsfluid frei von Alkalimetall-Chlorid, nachdem es in dem Gas-Flüssigkeitsabscheider (12) von dem anodischen Elektrolyt getrennt und entnommen wurde,
eine Eingangsleitung (15) für Lösungsfluid frei von Alkalimetall-Chlorid zum Zuführen von Lösungsfluid zu dem Auflösen von Chlorgas,
eine Eingangsleitung (7) für Ausgangswasser zum Zuführen von Ausgangswasser frei von Alkalimetall-Chlorid zu der Kathodenkammer (3), und
eine Entnahmeleitung (9) für alkalisches elektrolysiertes Wasser, um in der Kathodenkammer erzeugtes alkalisches elektrolysiertes Wasser aus der Kathodenkammer (3) zu entnehmen,
wobei die Höhe des oberhalb der Anodenkammer (2) angeordneten Gas-Flüssigkeitsabscheiders (12) größer gehalten wird als die Höhe des Vorratsbehälters (8) für alkalisches elektrolysiertes Wasser und die Entnahmeleitung (9) für elektrolysiertes Wasser stromabwärts von der Kathodenkammer (3) angeordnet ist.

7. Vorrichtung zum Erzeugen von elektrolysiertem Wasser gemäß Anspruch 6, wobei der pH des Alkalimetall-Chlorid-freien, sauren elektrolysierten Wassers eingestellt wird durch Hinzufügen von elektrolytisch hergestelltem saurem elektrolysiertem Wasser mit einer geregelten Flussrate zu dem Auflöser für Chlorgas, in welchem das Alkalimetall-Chlorid-freie, saure elektrolysierte Wasser hergestellt wird, indem das Gas, das in dem Gas-Flüssigkeitsabscheider von dem anodischen Elektrolyt getrennt und gesammelt wurde, mit Lösungsfluid in Kontakt gebracht wird, um es darin zu lösen.

8. Vorrichtung zum Erzeugen von elektrolysiertem Wasser, gemäß Anspruch 6 oder Anspruch 7, wobei Alkalimetall-Chlorid-freies, saures elektrolysiertes Wasser hergestellt wird durch Entnehmen von in dem Vorratsbehälter für anodischen Elektrolyten entwickeltem chlorhaltigem Gas, Mischen mit in der Anodenkammer entwickeltem chlorhaltigem Gas und Inkontaktbringen der gemischten Gase mit dem Lösungsfluid, um sie darin zu lösen.

9. Vorrichtung zum Erzeugen von elektrolysiertem Wasser, gemäß einem der Ansprüche 6 bis 8, wobei ein Teil des zugeführten Rohwassers in der Eingangsleitung zum Zuführen von Alkalimetall-Chlorid-freiem Rohwasser zu der Kathodenkammer abgezweigt und verbunden wird mit der Eingangsleitung zum Zuführen von Lösungsfluid zu dem Auflöser für Chlorgas, und abgezweigtes Rohwasser als Lösungsfluid verwendet wird.

## Revendications

1. Procédé de génération d'eau électrolysée, comprenant les étapes:
un électrolyte anodique comprenant une solution aqueuse contenant un chlorure d'un métal alcalin en solution est amené et mis en circulation, à partir d'un réservoir de stockage (10) d'électrolyte anodique qui retient l'électrolyte anodique, vers une chambre anodique (2) d'une cellule à deux compartiments (1) subdivisée par une membrane échangeuse de cations (4) en deux chambres, à savoir une chambre anodique (2) logeant une anode (5) et une chambre cathodique (3) logeant une cathode (6), ladite cathode (6) étant fabriquée en matériaux poreux et étant disposée de façon à être attachée à proximité de ladite membrane échangeuse de cations (4), et la contre-pression de ladite chambre anodique (2) étant rendue plus grande que celle de ladite chambre cathodique (3),
de l'eau brute, exempte de chlorure de métal alcalin, est amenée à la chambre cathodique (3), ladite eau brute exempte de chlorure de métal alcalin consistant en une eau douce préparée par élimination de l'ion Ca et de l'ion Mg contenus dans de l'eau de puits et de l'eau de ville ou dans une eau ayant subi un échange d'ions préparée par élimination additionnelle d'autres cation et anion, ou dans de l'eau pure préparée par élimination additionnelle de composants organiques,
et
une électrolyse est mise en oeuvre, dans laquelle
il y a production d'une eau électrolysée alcaline exempte de chlorure de métal alcalin au niveau de la chambre cathodique (3), et, simultanément, il y a production d'un gaz contenant du chlore au niveau de la chambre anodique (2),
après que le gaz est séparé et recueilli de l'électrolyte anodique, ledit gaz est amené à entrer en contact avec le fluide de dissolution exempt de chlorure de métal alcalin pour y être dissous, et
une eau électrolysée acide, exempte de chlorure de métal alcalin, est produite.

2. Procédé de génération d'eau électrolysée selon la revendication 1, dans lequel, quand le gaz séparé et recueilli de l'électrolyte anodique est mis en contact avec le fluide de dissolution pour être dissous, une eau électrolysée alcaline produite par électrolyse est ajoutée au fluide de dissolution à un débit régulé, pour ajuster le pH de l'eau électrolysée acide exempte de chlorure de métal alcalin.

3. Procédé de génération d'eau électrolysée selon la revendication 1 ou la revendication 2, dans lequel, après que le gaz contenant du chlore qui se dégage du réservoir de stockage d'électrolyte anodique est recueilli et mélangé au gaz contenant du chlore qui se dégage dans la chambre anodique, le mélange de gaz contenant du chlore est mis en contact avec le fluide de dissolution pour y être dissous, pour produire une eau électrolysée acide exempte de chlorure de métal alcalin.

4. Procédé de génération d'eau électrolysée selon l'une quelconque des revendications 1 à 3, dans lequel l'électrolyse est mise en oeuvre par application d'un électrolyte anodique dans lequel le chlorure de métal alcalin est dissous à raison de 10 % en poids ou plus.

5. Procédé de génération d'eau électrolysée selon l'une quelconque des revendications 1 à 4, dans lequel le temps de contact et de dissolution du gaz contenant du chlore généré par électrolyse, avec le fluide de dissolution, est de 0,05 seconde ou plus pour 1 ml du gaz.

6. Générateur d'eau électrolysée, destiné à produire simultanément une eau électrolysée acide exempte de chlorure de métal alcalin et une eau électrolysée alcaline exempte de chlorure de métal alcalin, grâce à une cellule (1) à deux compartiments, comprenant :
une cellule (1) à deux compartiments, subdivisée en deux compartiments, à savoir une chambre anodique (2) logeant une anode (5) et une chambre cathodique (3) logeant une cathode (6), par une membrane échangeuse de cations (4), ladite cathode (6) étant fabriquée en matériaux poreux et étant disposée de façon à être attachée à proximité de ladite membrane échangeuse de cations (4), et dans lequel, en cours d'utilisation, la contre-pression de ladite chambre anodique (2) est plus grande que celle de ladite chambre cathodique (3),
un réservoir de stockage (10) d'un électrolyte anodique destiné à retenir l'électrolyte anodique comprenant une solution aqueuse dans laquelle un chlorure de métal alcalin est dissous,
un circulateur (11), qui fait circuler l'électrolyte anodique se trouvant dans le réservoir de stockage (10) d'électrolyte anodique, vers la chambre anodique (2),
un tube de sortie (20) d'électrolyte anodique, pour évacuer de la chambre anodique (2) le gaz contenant du chlore qui se dégage dans la chambre anodique (2), et l'électrolyte anodique contenant le gaz dissous,
un séparateur gaz-liquide (12), qui sépare le gaz contenant du chlore provenant du tube de sortie (20) d'électrolyte anodique, et l'électrolyte anodique contenant le gaz dissous,
un dissolveur (18) de chlore gazeux, destiné à produire une eau électrolysée acide exempte de chlorure de métal alcalin, par mise en contact, avec le fluide de dissolution exempt de chlorure de métal alcalin, du gaz après qu'il a été séparé et recueilli de l'électrolyte anodique au niveau du séparateur gaz-liquide (12),
un tube d'entrée (15) de fluide de dissolution exempt de chlorure de métal alcalin, pour amener le fluide de dissolution au dissolveur de chlore gazeux,
un tube d'entrée (7) d'eau brute, pour amener de l'eau brute exempte de chlorure de métal alcalin à la chambre cathodique (3), et
un tube de sortie (9) d'eau électrolysée alcaline, pour évacuer de la chambre cathodique (3) l'eau électrolysée alcaline produite dans la chambre cathodique,
la hauteur du séparateur gaz-liquide (12) situé au-dessus de la chambre anodique (2) étant maintenue supérieure à la hauteur du réservoir de stockage d'eau électrolysée alcaline (8) et du tube de sortie d'eau électrolysée (9) situé en aval de la chambre cathodique (3).

7. Générateur d'eau électrolysée selon la revendication 6, dans lequel le pH de l'eau électrolysée acide exempte de chlorure d'un métal alcalin est régulé par ajout dans le dissolveur de chlore gazeux d'une eau électrolysée alcaline produite par électrolyse, à un débit contrôlé, l'eau électrolysée acide exempte de chlorure de métal alcalin étant produite avec le gaz séparé et recueilli de l'électrolyte anodique au niveau du séparateur gaz-liquide, ledit gaz étant amené à entrer en contact avec le fluide de dissolution pour y être dissous.

8. Générateur d'eau électrolysée selon la revendication 6 ou la revendication 7, dans lequel l'eau électrolysée acide exempte de chlorure de métal alcalin est produite en recueillant le gaz contenant du chlore qui se dégage dans le réservoir de stockage d'électrolyte anodique, en mélangeant ce gaz avec le gaz contenant du chlore qui se dégage dans la chambre anodique, et en mettant les gaz mélangés en contact avec le fluide de dissolution pour y être dissous.

9. Générateur d'eau électrolysée selon l'une quelconque des revendications 6 à 8, dans lequel une portion de l'eau brute dans le tube d'entrée servant à amener l'eau brute exempte de chlorure de métal alcalin à la chambre cathodique, est dérivée et reliée au tube d'entrée destiné à amener le fluide de dissolution dans le dissolveur de chlore gazeux, et l'eau brute dérivée est utilisée en tant que fluide de dissolution.
